# FASCICULE DE BREVET EUROPEEN

(11) **EP 3 532 596 B1**
(45) Date de publication et mention de la délivrance du brevet: **20.01.2021**
(21) Numéro de dépôt: 17797696.6
(22) Date de dépôt: 23.10.2017
(51) Int. Cl.: C12G 3/00, C12G 3/04

(54) **ETHANOL ALIMENTAIRE AMELIORE**
VERBESSERTES LEBENSMITTELTAUGLICHES ETHANOL
IMPROVED FOOD-GRADE ETHANOL

(30) Priorité: 27.10.2016 FR 1670637
(43) Date de publication de la demande: 04.09.2019
(73) Titulaire: Probst, Laurent, 94140 Alfortville (FR)
(72) Inventeur: Probst, Laurent, 94140 Alfortville (FR)
(74) Mandataire: Hege, Frédéric
(86) Numéro de dépôt international: PCT/FR2017/052914
(87) Numéro de publication internationale: WO 2018/078267

(56) Documents cités:
- FR-A1- 2 178 983
- JP-A- S6 363 777
- KR-A- 20120 135 748
- US-A- 5 961 707
- DATABASE WPI Week 198006 1980 Thomson Scientific, London, GB; AN 1980-10481C XP002770360, & JP S55 1035 B (HAYAKAWA N) 11 janvier 1980 (1980-01-11)

## Description

La présente invention se situe dans le domaine de la cuisine. Elle concerne plus particulièrement un éthanol alimentaire amélioré pouvant être utilisé dans différentes recettes alimentaires, en particulier comme moyen de cuisson maîtrisé pour en faire un spectacle pour le convive ou le client. L'éthanol est alors répandu dans un réceptacle pour s'y consumer et dégager la chaleur correspondante, ou encore l'éthanol est répandu sur l'aliment, par exemple une crème, pour que la combustion de l'éthanol produise l'effet de la crème brûlée.

Il est connu, par une demande de brevet déposé par le demandeur, n°EP1 635 683, un procédé de cuisson à l'éthanol pur. Quand on enflamme un éthanol pur, en particulier de titre supérieur à 80°, tout d'abord il s'enflamme à température ambiante, sans nécessité de préchauffage, et il se consume intégralement. Cet effet est recherché pour des plats comme une crème brûlée, où on cherche à faire une caramélisation en produisant une flamme à sa surface, ou encore une viande ou un poisson ou une tarte flambée, pour laquelle on souhaite ajouter un arôme après la cuisson ; on prend alors un éthanol dans lequel on ajoute un arôme, on le répand à la surface du plat, on l'enflamme à température ambiante, et l'éthanol se consume intégralement, laissant la seule arôme à la surface du mets, avec un effet grill sur la surface du mets.

Toutefois, pour obtenir un tel résultat, il faut éviter de laisser l'éthanol à l'air et à la surface du mets trop longtemps, sinon il se dilue avec l'eau du mets, ou l'humidité de l'air ambiant. Après seulement quelques minutes, l'éthanol s'est dilué, il devient plus difficile à enflammer, et il ne se consume plus entièrement, l'eau présente dans l'éthanol éteignant la flamme avant que tout l'éthanol ne se soit consumé. Or quand on veut par exemple proposer un tel procédé à une tablée de plusieurs personnes, il peut être intéressant de déposer l'éthanol sur les plats de chacun, puis à les enflammer en même temps. Il se passe donc facilement une ou deux minutes entre le premier dépôt d'éthanol et son inflammation.

Or de telles possibilités sont très appréciées, et correspondent à une demande des utilisateurs. En effet des clients d'un restaurant apprécient de voir leur crème brûlée s'enflammer et se former devant leurs yeux, ou de voir un œuf entrain de cuire dans un coquetier par une flamme visible ou encore de voir une brochette cuire devant leurs yeux. Et ils apprécient, quand ils sont à plusieurs, de voir ces spectacles se produire en même temps sur toutes les assiettes de la tablée

FR2178983 A1 (GEN FOODS CORP [US]) décrit une composition utilisée pour flamber une composition alimentaire avec un éthanol titré à 56.2° mélangé à de la dextrine expansée de manière à obtenir une poudre. JPS551035 B (HAYAKAWA N) décrit un alcool de type sake ou whisky sous forme solide, le dit alcool étant mélangé avec du dibenzal sorbitol de l'hydroxypropyl cellulose. US5961707 A (MOTHES HELMUT [DE] ET AL) décrit de l'éthanol sous forme pulvérulente, titré à 87%, la poudre étant formée par mélange avec de l'amidon hydrolysé. JPS6363777 A (ASAHI DENKA KOGYO KK) décrit de l'éthanol solidifié par mélange avec de 20-70 % en poids d'éthanol avec 80-30 % en poids d'un polyalcool tel que le sorbitol. KR20120135748 A (SAMSUNG FINE CHEMICALS CO LTD [KR]) décrit un soju (titré à 30° d'éthanol) dans lequel est ajouté de l'hydroxypropylmethyl cellulose.

L'inventeur s'est aperçu qu'en ajoutant une faible quantité d'un hydroxy-alkyl-cellulose à l'éthanol, il se diluait nettement moins vite dans l'eau environnant, et pour un éthanol suffisamment concentré au départ, il devient possible d'enflammer l'éthanol plusieurs minutes après l'avoir déposé, même sur un mets très humide. Pour un éthanol de titre compris entre 80 et 85°, l'éthanol ne se consume pas complètement à la température ambiante, et il vaut mieux éviter cette plage de titre.

La présente invention a pour objet de pallier au moins en partie à ces inconvénients. A cet effet, elle propose un éthanol de titre compris entre 85° et 99° comportant un Hydroxy-Alkyl-Cellulose (HAC), ledit HAC étant dosé pour obtenir une viscosité inférieure 100 Pa•s.

Grâce à ces dispositions, l'éthanol selon l'invention peut facilement être déposé de manière uniforme à la surface par exemple d'une crème brûlée, ou d'une viande ou poisson cuits. La quantité d'HAC à utiliser pour ne pas dépasser la viscosité dynamique revendiquée dépend fortement du type d'HAC utilisé, mais l'homme du métier sait parfaitement doser l'HAC, en utilisant des abaques, ou par quelques essais simples. Par ailleurs, en proposant un éthanol de titre compris entre 85° et 99°, on s'assure d'une part que l'éthanol s'enflamme, malgré l'ajout d'un faible pourcentage d'HAC, et d'autre part on évite des procédés de fabrication de l'éthanol trop onéreux visant à enlever le dernier pourcent d'eau.

Selon d'autres caractéristiques :
- ledit HAC peut être un Hydroxy-Propyl-Cellulose (HPC), ce produit donnant des résultats particulièrement satisfaisants,
- le dosage en Hydroxy-Propyl-Cellulose peut être d'au moins 1g par litre d'éthanol, ce dosage permettant d'obtenir l'effet souhaité de non dilution de l'alcool avec de l'eau.

La présente invention concerne encore l'utilisation d'un éthanol selon l'invention comme combustible pour réaliser la cuisson d'un aliment, ou d'une partie d'un aliment. Cela permet de prendre un peu de temps entre la mise en contact de l'éthanol avec l'aliment et/ou l'air ambiant, et son inflammation.

Selon d'autres caractéristiques :
- ladite cuisson peut s'opérer à proximité immédiate de la personne à qui est destiné ledit aliment, de sorte à utiliser la cuisson comme spectacle devant un convive ou un client assis à sa table pour un repas ; l'avantage de ce procédé est que l'éthanol peut être déposé tranquillement sur un mets d'un convive, puis sur les mets de chaque autre convive autour de la table, puis plusieurs serveurs peuvent s'organiser pour enflammer l'éthanol de tous les mets de la table en même temps,
- ledit aliment peut être un œuf disposé dans un récipient transparent, ledit éthanol étant disposé dans un réservoir à l'extérieure du récipient, permettant ainsi d'observer la cuisson de l'œuf,
- ledit aliment peut être une brochette, par exemple de viande ou de poisson, ledit éthanol étant disposé dans un demi cylindre, et la brochette étant posée sur ledit demi cylindre, permettant une cuisson propre et inodore,
- ladite cuisson peut consister en la caramélisation d'une crème pour en faire une crème brûlée ; on obtient également, de manière très surprenante, une couche de cellulose à la surface de la crème brûlée, et cette couche est imperméable à l'eau, et protège donc la crème brûlée de l'humidité ambiante ; la crème brûlée peut ainsi être servie plusieurs heures après la caramélisation, sans perdre le croustillant de la croûte formée, ce qui n'empêche nullement de la déguster immédiatement,
- ladite utilisation peut comprendre les étapes suivantes :
   - on dispose l'éthanol dans un contenant de type crayon,
   - on dépose l'éthanol sur un aliment à l'aide dudit crayon, selon un dessin particulier
   - on enflamme l'éthanol,
      cela permet de disposer de temps entre la dépose de l'éthanol sur l'aliment et inflammation de l'éthanol,
- ladite utilisation peut comprendre les étapes suivantes :
   - fabrication de perles
   - encapsulation de ces perles, par exemple en les entourant d'une algue
   - introduction des perles obtenues dans des crèmes glacées,
      permettant ainsi d'obtenir des perles non congelées aux températures de congélation de crèmes glacées, de l'ordre de -18°C.

La présente invention sera mieux comprise à la lecture de la description détaillée qui fait suite, en référence aux figures annexées dans lesquelles :
- La figure 1 illustre une assiette avec un mets, par exemple une viande
- La figure 2 illustre l'assiette de la fig. 1, avec déposition d'un éthanol selon l'invention à l'aide d'un crayon
- La figure 3 illustre l'assiette de la fig. 1, le mets étant entrain de flamber.

L'inventeur a cherché longtemps une solution pour les utilisateurs, convives ou clients de restaurants, par une cuisson qui se passe sous leurs yeux. Il connaissait la solution de l'éthanol titrant plus de 80°, qui permet une cuisson inodore et une combustion complète de l'éthanol. Toutefois cet éthanol impose d'aller très vite, sinon il se dilue avec l'eau environnant, et s'évapore très rapidement. Or dans certains cas on souhaite avoir un peu plus de temps.

L'inventeur a donc réalisé de nombreux essais, avec des produits variés. Il s'est aperçu qu'en ajoutant une faible quantité d'un Hydroxy-Alkyl-Cellulose à un éthanol titrant entre 85 et 99°, la dilution avec l'eau est très nettement plus lente, et l'évaporation est également ralentie.

L'inventeur s'est aperçu que pour obtenir un effet satisfaisant, et réduire suffisamment la dilution avec l'eau, la quantité d'HAC doit être d'au moins 1g d'HAC par litre d'éthanol. Par ailleurs, pour être utilisable de manière satisfaisante, l'éthanol doit avoir de préférence une viscosité dynamique inférieure à 100 Pa•s, valeur au-dessus de laquelle l'éthanol devient trop difficile à étaler. Ces viscosités peuvent être obtenues par exemple avec un HPC de masse moléculaire 100 000 g/mol dosé à 60 g/litre d'éthanol.

On peut aussi obtenir ce résultat avec un HPC de masse moléculaire 850 000 g/mol dosé à moins de 20 g/litre d'éthanol.

La valeur de la viscosité est mesurée « au repos », c'est à dire à une vitesse de cisaillement de 0,1 s⁻¹. Cette précision est importante du fait que la viscosité dynamique diminue quand la vitesse de cisaillement augmente. Dans le cadre de la présente demande de brevet, chaque fois qu'il est question d'une viscosité , il faut comprendre qu'il s'agit de la viscosité dynamique au repos, c'est à dire à une vitesse de cisaillement de 0,1 s⁻¹.

Selon le type d'HAC utilisé, la viscosité dynamique augmente plus ou moins vite pour une quantité ajoutée, et il est important pour l'application de se limiter à 100 Pa•s, sinon il devient très difficile de répandre l'éthanol obtenu, ou de le faire sortir d'un crayon. Il est même préférable de se limiter à 50 Pa•s pour obtenir un produit agréable à manipuler.

L'homme du métier sait parfaitement doser l'apport d'HAC pour obtenir une viscosité dynamique donnée.

On peut utiliser divers produits de la famille des Hydroxy-alkyl-Celluloses, tel qu'un HPC, mais aussi un Hydroxy-Ethyl-Cellulose, un Hydroxy-Ethyl-Methyl-Cellulose, ou encore un Hydroxy-Propyl-Methyl-Cellulose pour obtenir un effet similaire de ralentissement de l'absorption d'eau de l'éthanol obtenu.

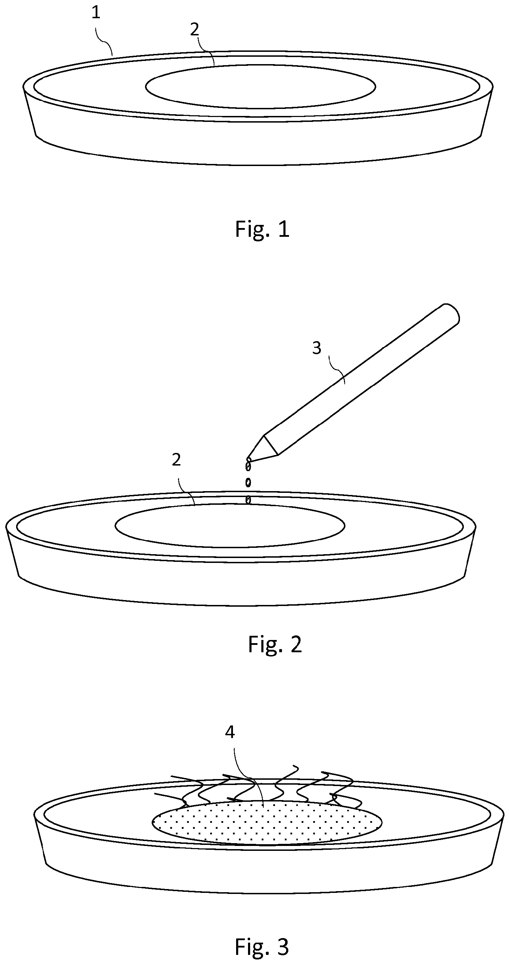

Un tel éthanol peut être aromatisé, puis utilisé pour cuire et dorer un poisson ou une viande. On peut disposer dans une assiette 1 ledit poisson 2 (voir fig. 1). Ensuite (fig. 2), à l'aide d'un crayon 3 contenant un éthanol selon l'invention, disposer ledit éthanol sur le poisson 2. On peut attendre que tout soit prêt, puis enflammer l'éthanol, et le voir brûler (fig. 3). On obtient un poisson avec une fine croute dorée 4 résultant de la grillade d'une fine couche de poisson.

On peut prendre par exemple un éthanol titrant à 95°, et ajouter une quantité de 20g/l d'HPC à 850 000g/mol. On peut disposer l'éthanol tranquillement alors que l'assiette est déposée devant le convive. Non seulement, l'éthanol selon l'invention ne se dilue pas par l'eau du mets, ou de l'air environnant, mais en plus, du fait de la quantité plus grande d'HPC utilisée, et de la propriété de l'HPC d'augmenter la viscosité de l'alcool, il reste en place sur le mets là où on l'a déposé, et ne coule que très peu. Cela est donc bien plus pratique à mettre en œuvre.

On peut donc attendre le moment opportun pour l'enflammer à l'aide d'un briquet ou d'une allumette. L'éthanol brûle alors, jusqu'à se consumer entièrement. On améliore aussi nettement la sécurité pour l'utilisateur, puisqu'on réduit nettement le risque que l'éthanol, notamment enflammé, s'échappe à l'occasion d'un renversement ou bousculement de l'assiette. Ce dernier avantage se retrouve pour sensiblement toutes les applications mentionnées dans la présente demande.

Il reste alors une fine couche de cellulose tout à fait invisible, et les arômes, ainsi qu'une fine couche de mets rôti sous l'effet de la chaleur dégagée par la combustion de l'éthanol.

L'invention peut être utilisée pour produire la caramélisation d'une crème pour en faire une crème brûlée. Quand on caramélise une crème avec un éthanol classique, par exemple de type rhum ou whisky à 40 degrés (ou 80 proof avec la mesure anglo-saxonne), on obtient une flamme qui dure moins de 10s. On est donc amené à mettre une quantité d'éthanol suffisante pour que la chaleur dégagée en moins de 10s permette d'obtenir une caramélisation satisfaisante. Avec un éthanol très pur, on obtient une flamme qui peut durer 30 à 40s, et l'éthanol est intégralement consumé. Cela présente l'avantage qu'il ne reste pas de goût d'éthanol sur la crème brûlée, et que la quantité d'éthanol nécessaire est bien plus faible.

Toutefois en utilisant de l'éthanol pur classique, il y a un risque que l'éthanol gicle, notamment si on bouscule le pot de crème pendant la combustion, ce qui peut toujours arriver. La caramélisation se fait donc le plus souvent en cuisine, où les conditions de sécurité peuvent être assurées par ailleurs, puis la crème brûlée est servie au convive ou au client. Elle doit toutefois être servie dans les 30 à 45 minutes au plus, sinon l'humidité de l'air ambiant fait ramollir la croûte, qui n'est alors plus croustillante, ce qui est assez gênant pour une crème brûlée. De plus, l'éthanol doit être enflammé immédiatement, dès qu'il est disposé sur la crème, sinon il a le temps de se diluer avec l'humidité de l'air ambiant, où avec l'eau de condensation déposé sur la crème, ou absorbée par des éclats de cassonade ou caramel.

Avec un éthanol selon l'invention, par exemple à 1,5 g/l d'HPC, l'éthanol n'absorbe pas l'humidité de l'air ambiant, ni l'eau de condensation ; en conséquence, on peut disposer l'éthanol sur la crème en cuisine, puis l'enflammer devant le convive.

En outre la croûte reste croustillante pendant plus de trois heures. La combustion laisse une couche de cellulose très fine, invisible, mais imperméable à l'humidité de l'air ambiant, et protège ainsi la crème brûlée, ce qui permet de préparer les crèmes brûlées à l'avance, ou de les faire brûler devant le convive, selon les besoins du moment.

L'éthanol selon l'invention peut également être utilisé dans un coquetier, l'œuf étant disposé dans un récipient transparent, et l'éthanol selon l'invention dans un réservoir disposé sous le récipient, par exemple un coquetier tel que ceux décrits dans les brevets français FR3004095 et FR3029094. L'éthanol selon l'invention ne se diluant pas par l'eau de l'air environnant, on peut le disposer dans le réservoir en cuisine, puis l'apporter à table, et ensuite seulement l'enflammer, par exemple en même temps pour plusieurs convives.

Pour des raisons similaires, il peut être utilisé avantageusement dans un dispositif à brochette, tel que celui décrit dans le brevet européen EP1635683.

Une autre utilisation de l'éthanol selon l'invention se situe dans le domaine des perles comestibles. Selon l'état de la technique, on sait encapsuler de l'éthanol fort, tel que le rhum ou le cointreau, après l'avoir gélifié. Un tel éthanol fort titre de l'ordre de 50° et son encapsulation se fait en l'entourant d'une algue, très chargée en eau. Le résultat en est une perle titrant de l'ordre de 20°, ou même 17°. On souhaite parfois intégrer de telles perles dans une crème glacée, pour produire un effet agréable sous la langue. Toutefois à la température de conservation de crèmes glacées, en congélateur à -18°C, un éthanol à 20° ou 17° gèle, et devient solide.

En prenant un éthanol selon l'invention, par exemple avec 10 ou 15 g d'HPC par litre d'éthanol, et en y ajoutant un arôme de rhum ou de cointreau, on peut l'encapsuler et obtenir des perles titrant à 40°. A de tels titres, l'éthanol reste liquide à -18°C, et l'effet recherché dans une crème glacée à cette température est parfaitement rendu.

Une autre utilisation de l'éthanol selon l'invention se situe dans le domaine des gels désinfectants pour les mains. A cette fin, l'éthanol peut être disposé dans un crayon. Lorsqu'un utilisateur désire se désinfecter les mains, il peut faire sortir l'éthanol selon l'invention et l'étaler sur ses mains. En se frottant les mains, il produit l'évaporation de l'éthanol et se désinfecte les mains. On peut bien sûr y associer un parfum au choix, qui reste sur les mains après l'évaporation de l'éthanol.

Encore une autre utilisation de l'éthanol selon l'invention est une utilisation pour parfumer un mets. Au lieu de saupoudrer ou asperger le mets avec un parfum, on introduit le parfum dans l'éthanol selon l'invention, on le dispose sur le mets, et on l'enflamme. Quand l'éthanol s'est consumé, le parfum est imprégné en surface sur le mets, et il ne reste aucune trace de l'éthanol.

## Revendications

1. Éthanol **caractérisé en ce que** son titre est compris entre 85° et 99°, et **en ce qu'**il comporte un Hydroxy-Alkyl-Cellulose, le dosage en Hydroxy-Alkyl- Cellulose étant limité de sorte à obtenir une viscosité dynamique inférieure à 100 Pa.s.

2. Éthanol selon la revendication précédente, dans lequel ledit Hydroxy-Alkyl-Cellulose est un Hydroxy-Propyl-Cellulose.

3. Éthanol selon la revendication précédente, dans lequel le dosage en Hydroxy-Propyl-Cellulose est d'au moins 1 g par litre d'éthanol.

4. Utilisation d'un éthanol selon l'une des revendications précédentes comme combustible pour réaliser la cuisson d'un aliment, ou d'une partie d'un aliment.

5. Utilisation selon la revendication précédente pour réaliser ladite cuisson à proximité immédiate de la personne à qui est destiné ledit aliment, de sorte à utiliser la cuisson comme spectacle.

6. Utilisation selon l'une des revendications 4 ou 5, pour réaliser la cuisson d'un œuf dans un récipient, ledit éthanol étant disposé dans un réservoir à l'extérieur du récipient.

7. Utilisation selon l'une des revendications 4 ou 5, pour réaliser la cuisson d'une brochette, par exemple de viande ou de poisson, ledit éthanol étant disposé dans un demi cylindre, et la brochette étant posée sur ledit demi cylindre.

8. Utilisation selon l'une des revendications 4 ou 5, pour caraméliser une crème, et en faire une crème brûlée.

9. Utilisation selon l'une des revendications 4 ou 5, comprenant les étapes suivantes :
• on dispose l'éthanol dans un contenant de type crayon,
• on dispose l'éthanol sur un aliment à l'aide dudit crayon, selon un dessin particulier
• on enflamme l'éthanol

10. Utilisation d'un éthanol selon l'une des revendications 1 à 3, comprenant les étapes suivantes :
• fabrication de perles
• encapsulation de ces perles, par exemple en les entourant d'une algue
• introduction des perles obtenues dans des crèmes glacées.

11. Utilisation d'un éthanol selon l'une des revendications 1 à 3, comprenant les étapes suivantes :
• on dispose l'éthanol dans un contenant de type crayon,
• on étale l'éthanol sur les mains de l'utilisateur à l'aide dudit crayon, afin de les désinfecter.

12. Utilisation d'un éthanol selon l'une des revendications 1 à 3, comprenant les étapes suivantes :
• on mélange un parfum dans ledit éthanol,
• on dispose ledit éthanol sur un mets,
• on enflamme l'éthanol et on le laisse se consumer entièrement.

## Patentansprüche

1. Ethanol, **dadurch gekennzeichnet, daß** sein Titer zwischen 85° und 99° liegt und daß es eine Hydroxy-Alkyl-Cellulose enthält, wobei die Dosierung der Hydroxy-Alkyl-Cellulose so begrenzt ist, daß eine dynamische Viskosität von weniger als 100 Pa-s erhalten wird.

2. Ethanol nach dem vorstehenden Anspruch, wobei die Hydroxy-Alkyl-Cellulose eine Hydroxy-Propyl-Cellulose ist.

3. Ethanol nach dem vorstehenden Anspruch, wobei der Anteil von Hydroxy-Propyl-Cellulose mindestens 1 g pro Liter Ethanol beträgt.

4. Verwendung eines Ethanols gemäß einer der vorstehenden Ansprüchen als Brennstoff zum Kochen eines Lebensmittels oder eines Teils eines Lebensmittels.

5. Verwendung gemäß dem vorstehenden Anspruch zur Durchführung des Kochens in unmittelbarer Nähe der Person, für die das Essen bestimmt ist, um das Kochen als Show zu verwenden.

6. Verwendung nach einem der Ansprüche 4 oder 5 zur Durchführung des Kochens eines Eies in einem Behälter, wobei das Ethanol in einem Reservoir außerhalb des Behälters angeordnet ist.

7. Verwendung nach einem der Ansprüche 4 oder 5 zum Kochen eines Spießes, z.B. von Fleisch oder Fisch, wobei das Ethanol in einem Halbzylinder angeordnet ist und der Spieß auf den Halbzylinder gelegt wird.

8. Verwendung nach einem der Ansprüche 4 oder 5 zum Karamellisieren einer Creme und zur Herstellung einer Crème brûlée daraus.

9. Verwendung gemäß einem der Ansprüche 4 oder 5, umfassend die folgenden Schritte:
• das Ethanol wird in einen bleistiftartigen Behälter angeordnet,
• das Ethanol wird mit dem Bleistift nach einem bestimmten Muster auf ein Lebensmittel aufgetragen
• das Ethanol wird entzündet

10. Verwendung eines Ethanols nach einem der Ansprüche 1 bis 3, umfassend die folgenden Schritte:
• Herstellung von Perlen
• Einkapselung dieser Perlen, zum Beispiel durch Umgeben mit Algen
• Einführung dieser Perlen in Eiscremes.

11. Verwendung eines Ethanols nach einem der Ansprüche 1 bis 3, umfassend die folgenden Schritte:
• das Ethanol wird in einen bleistiftartigen Behälter angeordnet,
• das Ethanol wird mit diesem Stift auf die Hände des Benutzers aufgetragen, um sie zu desinfizieren.

12. Verwendung eines Ethanols nach einem der Ansprüche 1 bis 3, umfassend die folgenden Schritte:
• ein Parfüm wird in das Ethanol gemischt,
• das Ethanol wird auf einen Teller angeordnet,
• das Ethanol wird angezündet und vollständig ausbrennen gelassen.

## Claims

1. Ethanol **characterized in that** its alcohol by volume is between 85° and 99°, and **in that** it comprises a Hydroxy-Alkyl-Cellulose, the dosage of Hydroxy-Alkyl-Cellulose being limited so as to obtain a dynamic viscosity of less than 100 Pa•s.

2. Ethanol according to the previous claim, wherein said Hydroxy-Alkyl-Cellulose is a Hydroxy-Propyl-Cellulose.

3. Ethanol according to the previous claim, wherein the dosage of Hydroxy-Propyl-Cellulose is at least 1 g per liter of ethanol.

4. Use of an ethanol according to one of the previous claims as a fuel for cooking a foodstuff, or part of a foodstuff.

5. Use according to the previous claim for carrying out said cooking in close proximity to the person for whom said food is intended, so as to use the cooking as a show.

6. Use according to one of claims 4 or 5, for cooking an egg in a container, said ethanol being disposed in a tank outside the container.

7. Use according to one of claims 4 or 5 for cooking a skewer, for example meat or fish, wherein the ethanol is arranged in a half cylinder and the skewer is placed on said half cylinder.

8. Use according to one of claims 4 or 5, to caramelize a cream and make it into a crème brûlée.

9. Use according to one of claims 4 or 5, comprising the following steps:
• ethanol is placed in a pencil-type container,
• ethanol is placed on a food using said pencil, following a particular drawing
• ethanol is ignited

10. Use of an ethanol according to one of claims 1 to 3, comprising the following steps:
• pearls making
• encapsulation of these pearls, for example by surrounding them with an algae
• introduction of the obtained pearls into ice creams.

11. Use of an ethanol according to one of claims 1 to 3, comprising the following steps:
• placing the ethanol in a pencil-type container,
• the ethanol is spread on the user's hands with the pencil, in order to disinfect them.

12. Use of an ethanol according to one of claims 1 to 3, comprising the following steps :
• mixing a perfume into said ethanol,
• placing said ethanol on a dish,
• ignition of the ethanol and leaving it to burn out completely.
